# EUROPÄISCHE PATENTSCHRIFT

(11) **EP 0 692 002 B1**
(45) Veröffentlichungstag und Bekanntmachung des Hinweises auf die Patenterteilung: **27.12.1996**
(21) Anmeldenummer: 94911925.9
(22) Anmeldetag: 18.03.1994
(51) Int. Cl.: C09B 47/067, C07D 487/22, G11B 7/24

(54) **VERFAHREN ZUR HERSTELLUNG VON NAPHTALOCYANINEN**
METHOD OF PREPARING NAPHTHALOCYANINES
PROCEDE DE PREPARATION DE NAPHTALOCYANINES

(30) Priorität: 30.03.1993 DE 4310371
(43) Veröffentlichungstag der Anmeldung: 17.01.1996
(73) Patentinhaber: BASF AKTIENGESELLSCHAFT, 67056 Ludwigshafen (DE)
(72) Erfinder: KIPPER, Juergen, D-76137 Karlsruhe (DE); ALBERT, Bernhard, D-67133 Maxdorf (DE)
(86) Internationale Anmeldenummer: EP9400863
(87) Internationale Veröffentlichungsnummer: WO9422960

(56) Entgegenhaltungen:
- EP-A- 0 433 220
- GB-A- 2 168 372
- GB-A- 2 200 650
- JOURNAL OF THE CHEMICAL SOCIETY,PERKIN TRANSACTIONS I 1988 , LETCHWORTH(GB) Seiten 2453 - 2458 M.J COOK ET AL 'octa-alkoxy Phthalocyanine and Naphthalocyanine Derivatives:Dyes with Q-Band Absorption in the far red or near infrared' in der Anmeldung erwähnt

## Beschreibung

Die vorliegende Erfindung betrifft ein neues Verfahren zur Herstellung von achtfach durch Alkoxygruppen substituierten metallfreien oder metallhaltigen Naphthalocyaninen durch Veretherung von 1,4-Dihydroxy-2,3-dicyanonaphthalin, Bildung des metallfreien Naphthalocyanins und gegebenenfalls anschließender Metallisierung.

In der EP-A-433 220 ist die Herstellung von metallfreien und metallhaltigen Naphthalocyaninen, ausgehend von 1,4-Dihydroxy-2,3-dicyanonaphthalinen beschrieben. Weiterhin ist aus der GB-A-2 200 650 die Herstellung dieser Naphthalocyanine ausgehend von 1,4-Dialkoxy-2,3-dicyanonaphthalinen bekannt. Schließlich sind in J. Org. Chem. 29, 3591, 1964, und in J. Chem. Soc., Perkin Trans. I, 1988, 2453, einzelne Teilschritte beschrieben. Es hat sich jedoch gezeigt, daß die aus dem Stand der Technik bekannten Methoden die Naphthalocyanine nur in unbefriedigender Ausbeute und Reinheit liefern.

Aufgabe der vorliegenden Erfindung war es daher, ein neues Verfahren zur Herstellung von Naphthalocyaninen bereitzustellen, das auf einfache Weise durchgeführt werden kann und mittels dessen die Zielprodukte in hoher Ausbeute und Reinheit gewonnen werden können.

Es wurde nun gefunden, daß die Herstellung von Naphthalocyaninen der Formel I 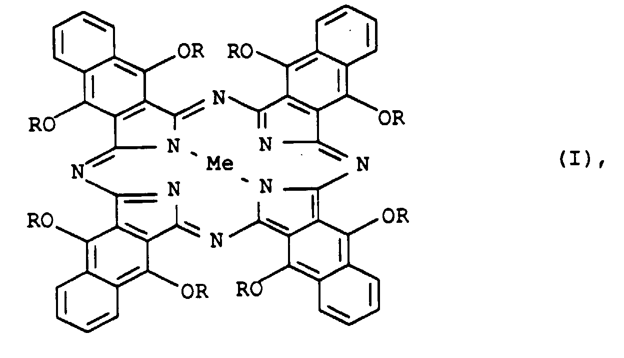 in der
- Me: zweimal Wasserstoff oder einen zweibindigen metallhaltigen Rest, der gegebenenfalls noch weitere Liganden aufweist, und
- R: jeweils C₁-C₂₀-Alkyl, das gegebenenfalls durch 1 bis 3 Sauerstoffatome in Etherfunktion unterbrochen ist, oder C₄-C₂₀-Alkenyl bedeuten,
durch Veretherung von 1,4-Dihydroxy-2,3-dicyanonaphthalin, Bildung des metallfreien Naphthalocyanins und gegebenenfalls anschließender Metallisierung vorteilhaft gelingt, wenn man
a) in einem 1. Schritt 1,4-Dihydroxy-2,3-dicyanonaphthalin in Gegenwart eines Verdünnungsmittels und einer Base mit einem Alkylierungsmittel der Formel II

   R-X (II),

   in der X Brom oder den Rest RO-SO₂O bedeutet und R jeweils die obengenannte Bedeutung besitzt, im Molverhältnis 1:2 bis 1:3 umsetzt, das Reaktionsgemisch auf Wasser gießt, das als Niederschlag anfallende Dicyanonaphthalin der Formel III 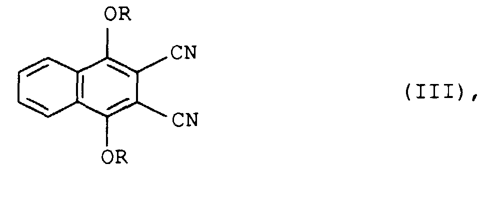 in der R jeweils die obengenannte Bedeutung besitzt, absaugt, wäscht und trocknet, dann
b) in einem 2. Schritt in Gegenwart eines Alkohols mittels eines Alkalialkoholats in das Naphthalocyanin der Formel I, in der Me zweimal Wasserstoff bedeutet, überführt, wobei man ein Molverhältnis Alkalialkoholat:Dicyanonaphthalin III von 1:2,5 bis 2,5:1 anwendet, und gegebenenfalls anschließend
c) in einem 3. Schritt das resultierende metallfreie Naphthalocyanin durch Umsetzung mit einem Metallsalz in Gegenwart eines Alkohols in das metallhaltige Naphthalocyanin der Formel I, in der Me einen metallhaltigen Liganden bedeutet, umwandelt, wobei Naphthalocyanin und Metallsalz im Molverhältnis von 1:1 bis 1:10 zur Reaktion gebracht werden.

Alle in den obengenannten Formeln auftretenden Alkyl- und Alkenylgruppen können sowohl geradkettig als auch verzweigt sein.

Die Reste R können dabei gleich oder verschieden sein.

Reste R sind z.B. Methyl, Ethyl, Propyl, Isopropyl, Butyl, Isobutyl, sec-Butyl, Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl, Isodecyl, Undecyl, Dodecyl, Tridecyl, 3,5,5,7-Tetramethylnonyl, Isotridecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), Tetradecyl, Pentadecyl, Hexadecyl, Heptadecyl, Octadecyl, Nonadecyl, Eicosyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3 Propoxypropyl, 2- oder 3-Butoxypropyl, 2- oder 4-Methoxybutyl, 2- oder 4-Ethoxybutyl, 2- oder 4-Propoxybutyl, 2- oder 4-Butoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,8-Trioxadecyl, 3,6, 9-Trioxaundecyl, Butenyl, But-3-en-1-yl, Pentenyl, Pent-4-en-1-yl, 3-Methyl-but-3-en-1-yl, Hexenyl, Heptenyl, Octenyl, Nonenyl, Decenyl, Undecenyl, Undec-10-en-1-yl, Dodecenyl, Tridecenyl, Tetradecenyl, Pentadecenyl, Hexedecenyl, Heptadecenyl, Octadecenyl, Nonadecenyl, oder Eicosenyl.

Als zweibindiger metallhaltiger Rest ist insbesondere Kupfer zu nennen.

Bevorzugt ist eine Verfahrensweise zur Herstellung von Naphthalocyaninen der Formel I, in der Me zweimal Wasserstoff oder Kupfer bedeutet.

Bevorzugt ist weiterhin eine Verfahrensweise zur Herstellung von Naphthalocyaninen der Formel I, in der R jeweils C₂-C₁₃-Alkyl oder C₄-C₁₁-Alkenyl und insbesondere C₂-C₈-Alkyl, bedeutet, wobei die Herstellung solcher Naphthalocyanine der Formel I, in der R jeweils C₄-Alkyl oder ein C₂/C₅-Alkyl-Gemisch bedeutet, besonders hervorzuheben ist.

Im 1. Schritt des erfindungsgemäßen Verfahrens wird 1,4-Dihydroxy-2,3-dicyanonaphthalin in Gegenwart eines Verdünnungsmittels mit einem Alkylierungsmittel II umgesetzt.

Das Molverhältnis 1,4-Dihydroxy-2,3-dicyanonaphthalin:Alkylierungsmittel II beträgt dabei 1:2 bis 1:3, vorzugsweise 1:2,2 bis 1:2,4.

Wie oben bereits ausgeführt, sind geeignete Alkylierungsmittel die entsprechenden Bromverbindungen (R-Br) oder Dialkylsulfate (ROSO₂OR), wobei bei niederen Alkylresten (Methyl und Ethyl) vorzugsweise die jeweiligen Dialkylsulfate zur Anwendung gelangen, während bei den anderen Resten vorzugsweise die jeweiligen Bromverbindungen Verwendung finden.

Der Alkylierungsschritt wird in Gegenwart eines Verdünnungsmittels und einer Base vorgenommen. Geeignete Verdünnungsmittel sind z.B. N,N-Dimethylformamid, N,N-Diethylformamid, N-Methylpyrrolidon oder Dimethylsulfoxid. Die Verwendung von N,N-Dimethylformamid ist bevorzugt.

Geeignete Basen sind z.B. Alkalicarbonate, wie Lithium-, Natrium- oder Kaliumcarbonat. Die Verwendung von Kaliumcarbonat ist bevorzugt.

Je mol 1,4-Dihydroxy-2,3-dicyanonaphthalin kommen in der Regel 1,5 bis 3 mol Base zur Anwendung. Bezogen auf das Gewicht von 1,4-Dihydroxy-2,3-dicyanonaphthalin wendet man in der Regel 400 bis 700 Gew.-% Verdünnungsmittel an.

Der 1. Schritt des erfindungsgemäßen Verfahrens wird üblicherweise bei einer Temperatur von 80 bis 120°C vorgenommen.

Er wird in der Regel so durchgeführt, daß man 1,4-Dihydroxy-2,3-dicyanonaphthalin und das Verdünnungsmittel vorlegt und zu diesem Gemisch Base und Alkylierungsmittel II gibt. Es ist jedoch auch möglich, diese Reihenfolge zu vertauschen und beispielsweise 1,4-Dihydroxy-2,3-dicyanonaphthalin, Verdünnungsmittel und Alkylierungsmittel II vorzulegen und dazu Base zu geben oder 1,4-Dihydroxy-2,3-dicyanonaphthalin, Verdünnungsmittel und Base vorzulegen und dazu Alkylierungsmittel II zu geben. Das resultierende Gemisch wird dann unter Rühren 4 bis 10 Stunden bei der obengenannten Temperatur gehalten.

Die weitere Aufarbeitung besteht darin, das Reaktionsgemisch auf Wasser zu gießen und das als Niederschlag anfallende Dicyanonaphthalin der Formel III abzusaugen, zu waschen und zu trocknen.

Es kann ohne weitere Reinigung direkt im 2. Schritt des erfindungsgemäßen Verfahrens zur Anwendung kommen, wobei diese Methode bevorzugt ist.

Die Herstellung des als Ausgangsprodukt verwendeten 1,4-Dihydroxy-2,3-dicyanonaphthalins ist z.B. in J. Org. Chem. 29, 3591, 1964, beschrieben.

Im 2. Schritt des erfindungsgemäßen Verfahrens wird das Dicyanonaphthalin der Formel III in Gegenwart eines Alkohols mittels eines Alkalialkoholats in das metallfreie Naphthalocyanin der Formel I übergeführt.

Dicyanonaphthalin III und Alkalialkoholat kommen dabei im Molverhältnis 1:2,5 bis 2,5:1, vorzugsweise 1:2 bis 2:1, zur Anwendung.

Geeignete Alkalialkoholate sind beispielsweise Lithium-, Natrium- oder Kaliumsalze von Methanol oder Ethanol. Die Verwendung der Natrium- oder Kaliumsalze ist bevorzugt, wobei Natriummethanolat oder Natriumethanolat besondere Bedeutung zukommt. Von besonderem Vorteil ist es weiterhin, die Alkalialkoholate in alkoholischer Lösung zuzugeben.

Geeignete Alkohole sind z.B. C₁-C₂₀-Alkanole, deren Alkylkette gegebenenfalls durch 1 bis 3 Sauerstoffatome in Etherfunktion unterbrochen ist, wie Methanol, Ethanol, Propanol, Isopropanol, Butanol, Isobutanol, sec-Butanol, Pentanol, Isopentanol, Neopentanol, tert-Pentanol, Hexanol, Heptanol, Octanol, Isooctanol, 2-Ethylhexanol, Nonanol, Decanol, oder C₄-C₂₀-Alkenole, wie But-3-en-1-ol, Pent-4-en-1-ol oder Undec-10-en-1-ol.

Die Verwendung von C₂-C₁₂-Alkanolen oder C₄-C₁₁-Alkenolen ist bevorzugt, wobei C₃-C₆-Alkanole besonders hervorzuheben sind.

Bezogen auf das Gewicht von Dicyanonaphthalin der Formel III kommen in der Regel 300 bis 1000 Gew.-% Alkohol zur Anwendung.

Der 2. Schritt des erfindungsgemäßen Verfahrens wird zweckmäßig so durchgeführt, daß man Alkalialkoholat, Alkohol und Dicyanonaphthalin III vorlegt und gegebenenfalls in einer Schutzgasatmosphäre, z.B Stickstoff oder Helium, unter Rühren auf eine Temperatur von 90 bis 160°C erhitzt.

Es ist auch möglich, eine Lösung beispielsweise von Natriummethanolat in Methanol zusammen mit dem Alkohol vorzulegen und dieses Gemisch zu erwärmen, um Methanol abzudestillieren. Nach dem Entfernen des Methanols kann dann das Dicyanonaphthalin III zugegeben werden.

Nach einer Reaktionsdauer von 0,25 bis 6 Stunden ist die Umsetzung im allgemeinen beendet. Man kann dann den Alkohol ganz oder teilweise abdestillieren (im letzten Fall mindestens bis zur Hälfte), zum Rückstand Methanol geben, das als Niederschlag anfallende metallfreie Naphthalocyanin der Formel I (Me - zweimal Wasserstoff) absaugen, mit Methanol nachwaschen und trocknen.

Das metallfreie Naphthalocyanin fällt in guter Reinheit an und kann ohne weitere Reinigung direkt weiter verwendet werden, z.B. für die Metallisierungsreaktion.

Am resultierenden Naphthalocyanin kann eine Umalkylierung (bis zu 100 %) der Reste R beobachtet werden. Während der Naphthalocyaninbildung können nämlich die am Naphthalinring vorhandenen OR-Reste teilweise gegen die Alkoholatreste des zur Anwendung kommenden Alkohols ausgetauscht werden.

Es ist auch möglich, am bereits gebildeten Octaalkoxynaphthalocyanin eine Umalkylierung vorzunehmen. Dies kann z.B. durch Behandlung des Octaalkoxynaphthalocyanins mit Natriummethanolat und einem Alkohol, dessen Alkoxyrest von den am Naphthalocyanin befindlichen Alkoxygruppen verschieden ist, geschehen.

Das metallfreie Naphthalocyanin der Formel I kann dann gegebenenfalls noch in einem 3. Schritt in ein metallhaltiges Naphthalocyanin umgewandelt werden. Dazu wird es in Gegenwart eines Alkohols mit einem Metallsalz umgesetzt. Naphthalocyanin und Metallsalz kommen dabei im Molverhältnis 1:1 bis 1:10, vorzugsweise 1:1 bis 1:1,2 zur Anwendung.

Bei der Durchführung des 3. Schrittes kann man das im 2. Schritt anfallende Reaktionsgemisch auch direkt, d.h. ohne Zwischenisolierung des metallfreien Naphthalocyanins, verwenden. Diese Methode ist bevorzugt.

Bezogen auf das Gewicht an metallfreiem Naphthalocyanin I werden in der Regel 1000 bis 2000 Gew.-% Alkohol verwendet.

Geeignete Metallsalze sind z.B. Kupfersalze, wie Kupfer(II)acetat, Kupfer(I)chlorid, Kupfer(II)chlorid, Kupfer(II)sulfat oder Kupfer(II)acetylacetonat. Die Verwendung von Kupfer(II)acetat oder Kupfer(I)- oder Kupfer(II)chlorid ist bevorzugt.

Geeignete Alkohole für diesen Schritt sind z.B. Propanol, Butanol oder Pentanol.

Wenn man die Metallisierung ohne Zwischenisolierung des metallfreien Naphthalocyanins durchführt, liegt bereits ein alkoholisches Reaktionsmedium vor. In diesem Fall kann man die Metallisierung direkt in diesem Medium durchführen oder gegebenenfalls noch mit weiterem Alkohol verdünnen.

In manchen Fällen kann es auch von Vorteil sein, die Metallisierung in Gegenwart von Basen, z.B. 1,4-Diazabicyclo[2.2.2]octan oder 1,5-Diazabicyclo[5.4.0]undec-5-en, durchzuführen.

Der 3. Schritt des erfindungsgemäßen Verfahrens wird zweckmäßig so durchgeführt, daß man ein Gemisch aus metallfreiem Naphthalocyanin I, Metallsalz, Verdünnungsmittel und gegebenenfalls Base unter Rühren auf eine Temperatur von 80 bis 160°C erhitzt. Nach einer Reaktionsdauer von 5 bis 20 Stunden ist die Umsetzung in der Regel beendet. Danach kann das Verdünnungsmittel ganz oder teilweise (im letzten Fall mindestens bis zur Hälfte) abdestilliert werden. Zum Rückstand kann man Petrolether geben und das metallhaltige Naphthalocyanin absaugen, waschen und trocknen.

Das erfindungsgemäße Verfahren, das sowohl in kontinuierlicher als auch diskontinuierlicher Arbeitsweise vorgenommen werden kann, ist einfach durchzuführen und liefert die Zielprodukte in hoher Ausbeute und Reinheit, wobei Ausbeute und Reinheit, verglichen mit den obengenannten Verfahren des Standes der Technik, im neuen Verfahren deutlich höher sind.

Bei den metallfreien oder metallhaltigen Naphthalocyaninen der Formel I handelt es sich um wertvolle im Infrarotbereich absorbierende Verbindungen, die beispielsweise in Druckfarben zur Anwendung gelangen können (siehe z.B. EP-A-553 614).

Die folgenden Beispiele sollen die Erfindung näher erläutern.

### I. Alkylierung

### Beispiel 1

42 g (0, 2 mol) 1,4-Dihydroxy-2,3-dicyanonaphthalin wurden in 200 ml N,N-Dimethylformamid (DMF) bei Raumtemperatur vorgelegt. Dazu gab man 55,4 g (0,44 mol) Dimethylsulfat und 60,8 g (0,44 mol) Kaliumcarbonat. Dann erwärmte man langsam auf 100°C und rührte 8 h bei dieser Temperatur. Danach wurde auf Wasser gefällt, abgesaugt, mit Wasser nachgewaschen und getrocknet.

Man erhielt 30 g 1,4-Dimethoxy-2,3-dicyanonaphthalin (Ausbeute: 64 %; Fp.: 190°C).

### Beispiel 2

21 g (0,1 mol) 1,4-Dihydroxy-2,3-dicyanonaphthalin, 100 ml Dimethylsulfoxid und 38,5 g (0,25 mol) Diethylsulfat wurden bei Raumtemperatur vorgelegt. Dazu wurden unter Rühren 38,6 g (0,28 mol) Kaliumcarbonat zugegeben. Anschließend wurde 10 h lang bei 100°C gerührt, danach auf Eiswasser gefällt, mit Wasser nachgewaschen und getrocknet.

Man erhielt 23,3 g 1,4-Diethoxy-2,3-dicyanonaphthalin (Ausbeute: 87 %; Fp.: 167°C).

### Beispiel 3

210 g (1 mol) 1,4-Dihydroxy-2,3-dicyanonaphthalin wurden in 1 l DMF zusammen mit 339 g (2,2 mol) Diethylsulfat bei Raumtemperatur vorgelegt. Zu dieser Mischung wurden vorsichtig 304 g (2,2 mol) Kaliumcarbonat gegeben. Es wurde langsam auf 100°C erhitzt und 6 h lang bei 100°C gerührt. Man fällte dann auf Wasser, wusch mit Wasser nach und trocknete.

Es wurden 231 g 1,4-Diethoxy-2,3-dicyanonaphthalin erhalten (Ausbeute: 87 %; Fp.: 167-169°C).

### Beispiel 4

105 g 1,4-Dihydroxy-2,3-dicyanonaphthalin, 145 g Pottasche und 500 ml DMF wurden vorgelegt. Zu diesem Gemisch wurden 155 g 4-Brombutan gegeben. Dann wurde unter Rühren auf 100°C erhitzt und 6 Stunden bei dieser Temperatur nachgerührt. Danach wurde das Reaktionsgemisch abgekühlt und in 2000 ml Eiswasser gegeben. Der Niederschlag wurde abgesaugt, mit Wasser gewaschen und bei 60°C unter vermindertem Druck getrocknet.

Man erhielt 120 g 1,4-Dibutoxy-2,3-dicyanonaphthalin (Ausbeute: 90 %; Fp.: 69-70°C).

### II. Naphthalocyaninbildung

### Beispiel 5

In 500 ml Ethanol wurden 3 g (0,13 mol) Natrium gelöst und 53,2 g (0,2 mol) 1,4-Diethoxy-2,3-dicyanonaphthalin zugegeben. Anschließend wurde 4 h unter Rückfluß gerührt. Ethanol wurde dann fast vollständig im Wasserstrahlvakuum abdestilliert (ca. 450 ml). Danach wurden 600 ml Methanol zum Reaktionsgemisch gegeben und 5 h lang bei Raumtemperatur gerührt. Das ausgefallene Produkt wurde abgesaugt, mit Methanol nachgewaschen und getrocknet.

Man erhielt 23 g Octaethoxynaphthalocyanin (Ausbeute: 43 %; λₘₐₓ: 856 nm; ε: 264 000 (in Toluol)).

### Beispiel 6

47 g (0,26 mol) 30 gew.-%ige methanolische Natriummethanolatlösung wurden zusammen mit 700 ml n-Propanol vorgelegt. Unter Normaldruck wurde das Methanol abdestilliert, bis die Siedetemperatur des Propanols (97°C) fast erreicht wurde. Nach dem Abkühlen gab man unter Stickstoffatmosphäre 106 g (0,4 mol) 1,4-Diethoxy-2,3-dicyanonaphthalin zu und rührte 3 h lang unter Rückfluß nach. Das Propanol wurde dann im Wasserstrahlvakuum bis auf einen Rest von ca. 50 ml abdestilliert, und man gab dann 1 1 Methanol zu. Nach 8 h Rühren bei Raumtemperatur wurde das ausgefallene Produkt abgesaugt, mit Methanol nachgewaschen und getrocknet.

Man erhielt 61,8 g eines Naphthalocyanins, das gemäß H-NMR zu 75 % transalkoxyliert wurde (R = 75 % Propyl und 25 % Ethyl) (Ausbeute: 53 %; λₘₐₓ: 858 nm; ε: 263 000 (in Toluol)).

### Beispiel 7

25 ml 30 gew.-%ige methanolische Natriummethanolatlösung wurden zusammen mit 500 ml Butanol vorgelegt. Unter Normaldruck wurde das Methanol abdestilliert, bis die Siedetemperatur des Butanols (117,8°C) fast erreicht wurde. Nach dem Abkühlen gab man unter Stickstoffatmosphäre 65 g 1,4-Dibutoxy-2,3-dicyanonaphthalin zu und rührte 2 h lang unter Rückfluß nach. Danach wurden 350 ml Butanol abdestilliert und 400 ml Methanol zugegeben. Nach 12 h Rühren bei Raumtemperatur wurde das ausgefallene Produkt abgesaugt, mit Methanol nachgewaschen und getrocknet.

Man erhielt 45 g Octabutoxynaphthalocyanin (Ausbeute: 70 %)

### Beispiel 8

47 g (0,26 mol) 30 gew.-%ige methanolische Natriummethanolatlösung wurden zu 1 1 n-Pentanol gegeben. Unter Normaldruck wurde Methanol abdestilliert, bis der Siedepunkt von Pentanol fast erreicht war (ca. 137°C). Nach Abkühlen wurden unter Stickstoffatmosphäre 106,4 g (0,4 mol) 1,4-Diethoxy-2,3-dicyanonaphthalin zugegeben und 3 h lang unter Rückfluß gerührt. Im Wasserstrahlvakuum wurde das Pentanol bis auf einen Rest von ca. 50 ml abdestilliert und 1 1 Methanol zugegeben. Nach 10 h Rühren bei Raumtemperatur wurde das ausgefallene Produkt abgesaugt, mit Methanol gewaschen und getrocknet.

Man erhielt 90,3 g eines teilweise transalkoxylierten Naphthalocyanins (R = 88 % Pentyl und 12 % Ethyl) (Ausbeute: 66 %; λₘₐₓ: 862 nm; ε: 260 000 (in Toluol)).

### Beispiel 9

18 g (0,1 mol) 30 gew.-%ige methanolische Natriummethanolatlösung wurden in 300 ml n-Hexanol vorgelegt. Unter Normaldruck wurde Methanol abdestilliert, bis eine Temperatur von 153°C erreicht war. Nach Abkühlen gab man 11,4 g (0,01 mol) eines Octaalkoxynaphthalocyanins (R = 70 % Propyl und 30 % Ethyl) zu und rührte 4 h unter Rückfluß. Im Wasserstrahlvakuum wurde Hexanol bis fast zur Trockene abdestilliert. Anschließend wurden 500 ml Methanol zugegeben. Nach 6 h Rühren bei Raumtemperatur wurde das ausgefallene Produkt abgesaugt, mit Methanol nachgewaschen und getrocknet.

Man erhielt 9,4 g Octahexyloxynaphthalocyanin (Ausbeute: 62 %; λₘₐₓ: 864 nm; ε: 208 000 (in Toluol)).

### III. Metallisierung

### Beispiel 10

45 g (0,035 mol) Octabutoxynaphthalocyanin wurden mit 7 g (0,039 mol) Kupfer(II)acetat-Monohydrat in 650 ml n-Butanol 10 h unter Rückfluß gerührt. Butanol wurde im Wasserstrahlvakuum abdestilliert, der Rückstand mit 700 ml Petrolether aufgerührt (1 h), abgesaugt, mit Petrolether gewaschen und getrocknet.

Man erhielt 42 g Kupfer-Octabutoxynaphthalocyanin (Ausbeute: 88 %; λₘₐₓ: 849 nm; ε: 235 000 (in Toluol)).

### IV. Naphthalocyaninbildung und Metallisierung im Eintopfverfahren

### Beispiel 11

5,4 g (0,03 mol) 30 gew.%ige methanolische Natriummethanolatlösung wurden bei Raumtemperatur unter Rühren in 180 ml n-Butanol vorgelegt. Dann wurde bei Normaldruck Methanol abdestilliert, bis der Siedepunkt von n-Butanol (117°C) im Reaktionsgefäß erreicht wurde. Die Lösung wurde auf 60°C abgekühlt und mit 16,1 g (0,05 mol) 1,4-Dibutoxy-2,3-dicyanonaphthalin versetzt. Anschließend wurde das Reaktionsgemisch unter Stickstoffatmosphäre 2 h unter Rückfluß gerührt, dann auf 60°C abgekühlt und bei dieser Temperatur mit 1,9 g (0,0094 mol) Kupfer(II)acetat-Monohydrat versetzt. Nach 15 h Rühren unter Rückfluß wurden 80 % des Gesamtvolumens an n-Butanol unter vermindertem Druck abdestilliert. Der Rückstand wurde mit Petrolether (180 ml) 2 h bei Raumtemperatur gerührt, abgesaugt und mit Petrolether gewaschen.

Man erhielt 13,4 g Kupfer-octa-butoxynaphthalocyanin (Ausbeute: 79,3 %; λₘₐₓ= 850 mm; ε= 180 000 (in Toluol)).

## Patentansprüche

1. Verfahren zur Herstellung von Naphthalocyaninen der Formel I 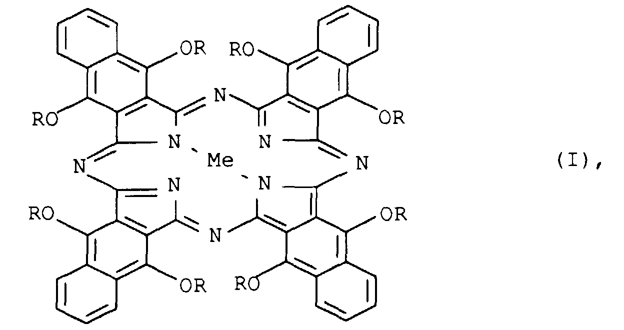 in der
Me zweimal Wasserstoff oder einen zweibindigen metallhaltigen Rest, der gegebenenfalls noch weitere Liganden aufweist, und
R jeweils C₁-C₂₀-Alkyl, das gegebenenfalls durch 1 bis 3 Sauerstoffatome in Etherfunktion unterbrochen ist, oder C₄-C₂₀-Alkenyl bedeuten,
durch Veretherung von 1,4-Dihydroxy-2,3-dicyanonaphthalin, Bildung des metallfreien Naphthalocyanins und gegebenenfalls anschließender Metallisierung,
dadurch gekennzeichnet, daß man
a) in einem 1. Schritt 1,4-Dihydroxy-2,3-dicyanonaphthalin in Gegenwart eines Verdünnungsmittels und einer Base mit einem Alkylierungsmittel der Formel II
R-X (II),
in der X Brom oder den Rest RO-SO₂O bedeutet und R jeweils die obengenannte Bedeutung besitzt, im Molverhältnis 1:2 bis 1:3 umsetzt, das Reaktionsgemisch auf Wasser gießt, das als Niederschlag anfallende Dicyanonaphthalin der Formel III 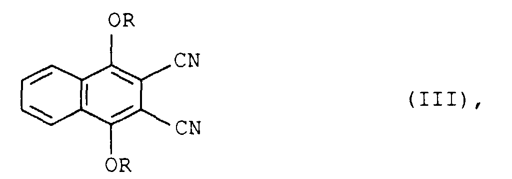 in der R jeweils die obengenannte Bedeutung besitzt, absaugt, wäscht und trocknet, dann
b) in einem 2. Schritt in Gegenwart eines Alkohols mittels eines Alkalialkoholats in das Naphthalocyanin der Formel I, in der Me zweimal Wasserstoff bedeutet, überführt, wobei man ein Molverhältnis Alkalialkoholat:Dicyanonaphthalin III von 1:2,5 5 bis 2,5:1 anwendet, und gegebenenfalls anschließend
c) in einem 3. Schritt das resultierende metallfreie Naphthalocyanin durch Umsetzung mit einem Metallsalz in Gegenwart eines Alkohols in das metallhaltige Naphthalocyanin der Formel I, in der Me einen metallhaltigen Liganden bedeutet, umwandelt, wobei Naphthalocyanin und Metallsalz im Molverhältnis von 1:1 bis 1:10 zur Reaktion gebracht werden.

2. Verfahren nach Anspruch 1, dadurch gekennzeichnet, daß Me zweimal Wasserstoff oder Kupfer bedeutet.

3. Verfahren nach Anspruch 1, dadurch gekennzeichnet, daß R jeweils C₂-C₁₃-Alkyl oder C₄-C₁₁-Alkenyl bedeutet.

4. Verfahren nach Anspruch 1, dadurch gekennzeichnet, daß R jeweils C₂-C₈-Alkyl bedeutet.

## Claims

1. A process for preparing naphthalocyanines of the formula I 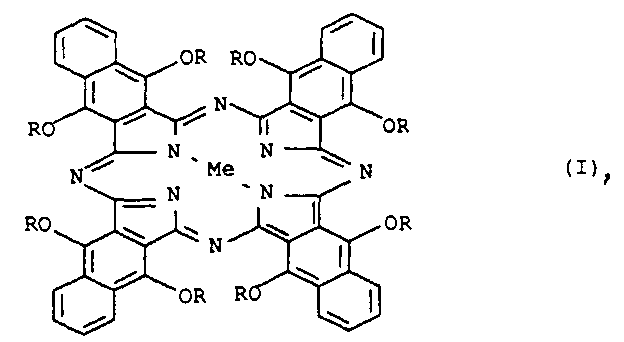 where
Me is twice hydrogen or a bivalent metal-containing radical with or without further ligands, and
R is in each case C₁-C₂₀-alkyl, which may be interrupted by from 1 to 3 oxygen atoms in ether function, or C₄-C₂₀-alkenyl,
by etherification of 1,4-dihydroxy-2,3-dicyanonaphthalene and formation of the metal-free naphthalocyanine with or without subsequent metallization,
characterized in that
a) in a first step the 1,4-dihydroxy-2,3-dicyanonaphthalene is reacted with an alkylating agent of the formula II
R-X (II),
where X is bromine or the radical RO-SO₂O and R is in each case as defined above, in a molar ratio of from 1:2 to 1:3 in the presence of a diluent and of a base, the mixture is poured onto water and the precipitated dicyanonaphthalene of the formula III 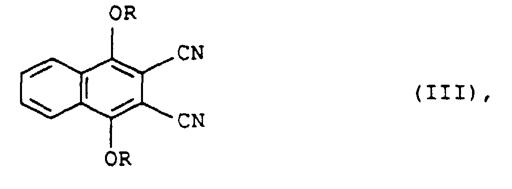 where R is in each case as defined above, is filtered off with suction, washed and dried, then
b) in a second step the dicyanonaphthalene of the formula III is converted with an alkali metal alkoxide into the naphthalocyanine of the formula I where Me is twice hydrogen in the presence of an alcohol, the molar ratio of alkali metal alkoxide:dicyanonaphthalene III being from 1:2.5 to 2.5:1, and optionally then
c) in a third step the resulting metal-free naphthalocyanine is converted by reaction with a metal salt in the presence of an alcohol into the metal-containing naphthalocyanine of the formula I where Me is a metal-containing ligand, the naphthalocyanine and the metal salt being reacted with each other in a molar ratio of from 1:1 to 1:10.

2. A process as claimed in claim 1, characterized in that Me is twice hydrogen or copper.

3. A process as claimed in claim 1, characterized in that R is in each case C₂-C₁₃-alkyl or C₄-C₁₁-alkenyl.

4. A process as claimed in claim 1, characterized in that R is in each case C₂-C₈-alkyl.

## Revendications

1. Procédé de préparation de naphtalocyanines de formule I 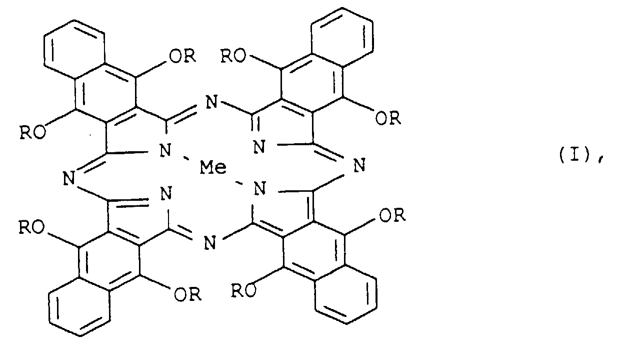 dans laquelle
Me représente deux fois un atome d'hydrogène ou un reste contenant un métal et formant deux liaisons, qui comporte éventuellement d'autres ligands encore, et
R représente chaque fois un reste alkyle en C₁-C₂₀, qui est éventuellement interrompu par 1 à 3 atomes d'oxygène en fonction éther, ou un reste alcényle en C₄-C₂₀,
par éthérification de 1,4-dihydroxy-2,3-dicyanonaphtalène, formation de la naphtalocyanine dépourvue de métal, suivie éventuellement de métallation,
caractérisé en ce que
a) dans une 1ère étape, on fait réagir du 1,4-dinydroxy-2,3-dicyanonaphtalène, en présence d'un diluant et d'une base, avec un agent d'alkylation de formule II
R-X (II)
dans laquelle X représente un atome de brome ou le reste RO-SO₂O et R a la signification donnée ci-dessus, dans un rapport molaire de 1:2 à 1:3, on verse le mélange réactionnel dans de l'eau, on filtre à la trompe, on lave et on sèche le dicyanonaphtalène de formule III obtenu en tant que précipité 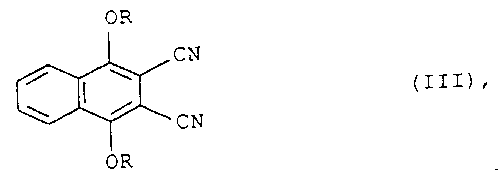 dans laquelle R a chaque fois la signification donnée ci-dessus, puis
b) dans une 2éme étape, on le transforme en la naphtalocyanine de formule I, dans laquelle Me représente deux fois un atome d'hydrogène, en présence d'un alcool, au moyen d'un alcoolate de métal alcalin, en appliquant un rapport molaire de l'alcoolate de métal alcalin au dicyanonaphtaléne III de 1:2,5 à 2,5:1, et éventuellement
c) dans une 3ème étape, on convertit ensuite la naphtalocyanine dépourvue de métal résultante, par réaction avec un sel métallique en présence d'un alcool, en la naphtalocyanine contenant un métal de formule I, dans laquelle Me représente un ligand contenant un métal, la naphtalocyanine et le sel métallique étant mis en réaction dans un rapport molaire de 1:1 à 1:10.

2. Procédé selon la revendication 1, caractérisé en ce que Me représente deux fois un atome d'hydrogène ou un atome de cuivre.

3. Procédé selon la revendication i, caractérisé en ce que R représente chaque fois un reste alkyle en C₂-C₁₃ ou alcényle en C₄-C₁₁.

4. Procédé selon la revendication 1, caractérisé en ce que R représente chaque fois un reste alkyle en C₂-C₈.
